# EUROPÄISCHE PATENTSCHRIFT

(11) **EP 0 811 662 B1**
(45) Veröffentlichungstag und Bekanntmachung des Hinweises auf die Patenterteilung: **05.09.2001**
(21) Anmeldenummer: 97108269.8
(22) Anmeldetag: 22.05.1997
(51) Int. Cl.: C09B 57/04, C07D 209/44, D06P 1/16

(54) **Isoindoleninamidfarbstoffe**
Isoindoleninamide dyes
Colorants à base d'isoindolenineamide

(30) Priorität: 04.06.1996 DE 19622356
(43) Veröffentlichungstag der Anmeldung: 10.12.1997
(73) Patentinhaber: DyStar Textilfarben GmbH & Co. Deutschland KG, 60318 Frankfurt am Main (DE)
(72) Erfinder: Lorenz, Manfred, Dr., 51061 Köln (DE)

(56) Entgegenhaltungen:
- EP-A- 0 684 289
- DE-A- 2 065 552
- FR-A- 1 470 022
- FR-A- 1 537 299
- FR-A- 2 307 008

## Beschreibung

Die Erfindung betrifft Isoindoleninamidfarbstoffe, Verfahren zu ihrer Herstellung sowie ihre Verwendung zum Färben von hydrophoben synthetischen Materialien.

Aus DE-A 16 70 748 sind bereits Isoindoleninamidfarbstoffe ähnlich denen der Formel (I) bekannt, die jedoch noch anwendungstechnische Nachteile aufweisen. Unter anwendungstechnischen Nachteilen sind beispielsweise zu geringe Zieh- oder Aufbauvermögen beim Färben von Polyester oder eine schlechte Lichtechtheit, insbesondere Heißlichtechtheit, also Eigenschaften wie sie bei Verwendung damit gefärbter Textilien auf dem Automobilsektor gefordert sind, zu verstehen.

Aus DE-A 2 065 552 sind bereits Oxazolyl-essigsäurederivate bekannt, die als Ausgangsstoffe für die Herstellung von Azo- und Methinfarbstoffen sowie für optische Aufheller geeignet sind.

EP-A 684 289 beschreibt Thiazol-isoindolenin Farbstoffe, deren Herstellung und Verwendung zum Färben von hydrophoben synthetischen Materialien.

Es wurden Isoindoleninamide gefunden, die der Formel I oder deren tautomeren Formen entsprechen, 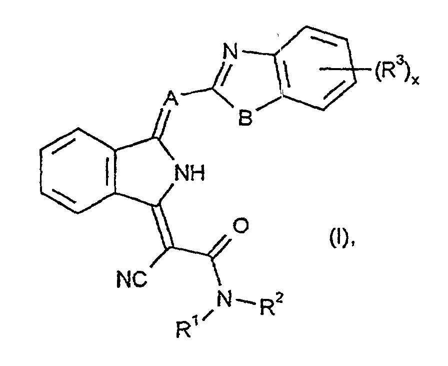 worin
- A: für N oder einen Cyanmethylenrest steht,
- B: für S, O oder NH steht,
- R¹: für einen gesättigten oder ungesättigten, aliphatischen oder cycloaliphatischen Rest mit 1 bis 12 C-Atomen, insbesondere 1 bis 10 C-Atomen, steht, der gegebenenfalls durch ein oder mehrere Sauerstoffatome unterbrochen ist und/oder gegebenenfalls durch einen oder mehrere gleiche oder verschiedene Substituenten aus der Reihe Alkoxy, Acyloxy, Halogen, CN, Aryl, insbesondere Phenyl substituiert ist,
- R²: Wasserstoff oder Aryl bedeutet oder eine Bedeutung annimmt, wie sie für R¹ angegeben ist, wobei R¹ und R² gleich oder verschieden sind, oder
- R¹ und R²: gemeinsam mit dem N-Atom, an das sie gebunden sind, einen heterocyclischen Ring bilden,
- R³: Halogen, insbesondere Cl, F und Br, C₁-C₄-Alkyl, ein gesättigter oder ungesättigter aliphatischer Oxyrest mit 1 bis 4 C-Atomen, insbesondere C₁-C₄-Alkoxy, der gegebenenfalls durch C₁-C₄Alkoxy substituiert ist, CN oder NO₂ bedeutet, und
- x: eine Zahl von 0 bis 4 bedeutet.

Geeignete Reste R¹ und R² sind z.B.: Methyl, Ethyl, n-Propyl, Allyl, n-Butyl, n-Pentyl, n-Hexyl, n-Heptyl, n-Octyl, n-Decyl, n-Dodecyl, 2-Acetoxy-ethyl, 2-Propionyloxy-ethyl, 2-Acetoxy-propyl, 2-Methoxy-ethyl, 2-Ethoxy-ethyl, 2-Butoxyethyl, 3-Methoxy-propyl, 3-Ethoxy-propyl, 3-Butoxy-propyl, 3-Allyloxy-propyl, 2-Ethyl-hexyl, 3-(2-Ethyl-hexyloxy)-propyl, Phenyl, Benzyl, Cyclohexyl. Als verzweigte Reste R¹ kommen vorzugsweise solche mit einer Methylseitenkette in Frage wie z.B.: iso-Butyl oder iso-Pentyl. Bevorzugte heterocyclische Ringe sind z.B. aliphatische 5-, 6- oder 7-Ringe.

Bevorzugte Verbindungen der Formel I sind solche, in denen die Reste R¹ und R² zusammen mit dem N-Atom, an das sie gebunden sind, die nachfolgend genannten sekundäre Amine bilden: Diethylamin, Dipropylamin, Diisopropylamin, Dibutylamin, Diisobutylamin, Methyl-butyl-amin, Methyl-(2-acetoxyethyl)-amin, Ethyl-(2-acetoxyethyl)-amin, Methyl-(2-acetoxypropyl)-amin, Bis-(2-acetoxyethyl)-amin, Bis-(2-acetoxypropyl)-amin, Pyrrolidin, Piperidin, 2-Methylpiperidin, 4-Methylpiperidin, Hexamethylenimin, Morpholin, Benzyl-methylamin, Cyclohexyl-methylamin.

Bevorzugte Reste R³ sind Chlor, Methyl, Methoxy und Ethoxy.

Besonders bevorzugte Farbstoffe der Formel (I) sind solche,
worin
- x: für 0 oder 1 steht.

Ganz besonders bevorzugte Farbstoffe sind solche, die der Formel (II) oder deren tautomeren Formen entsprechen, 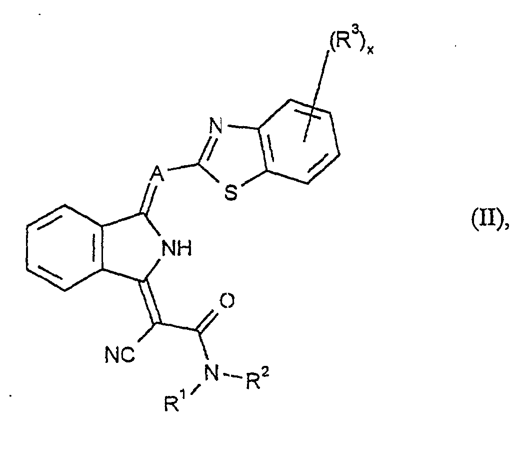 worin
- A, R¹ - R³: die obige Bedeutung besitzen, wobei x vorzugsweise für 0 steht.

Bevorzugte Isoindoleninamide der Formel (I) sind solche, worin A für N steht.

Sämtliche in dieser Anmeldung beschriebenen Formeln stellen zwar lediglich eine, sofern mehrere denkbar sind, tautomere Form der jeweiligen Verbindung(en) dar, stehen aber stellvertretend für alle denkbaren tautomeren Formen.

Weiterhin umfaßt ein durch eine Formel beschriebenes E- oder Z-Isomer, insbesondere im Hinblick auf die exocyclische(n) Doppelbindung(en), auch jeweils das andere Isomer. Dies gilt, sofern nicht ausdrücklich etwas anderes gesagt wird.

Die Erfindung betrifft weiterhin ein Verfahren zur Herstellung von Verbindungen der Formel (I), das dadurch gekennzeichnet ist, daß man eine Verbindung der Formel (III) 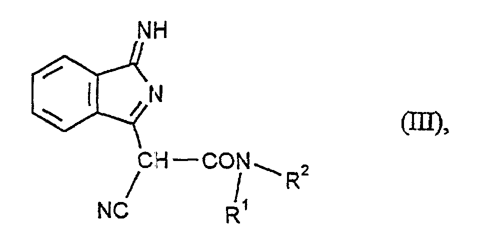 mit einer Verbindung der Formel (IV) 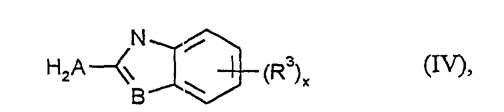 die in Form der Verbindungen der Formeln IVa oder IVb oder deren tautomeren Formen vorliegt, 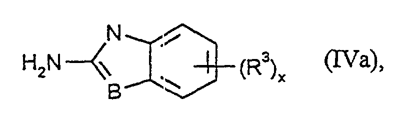 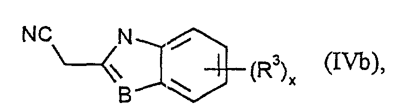 oder ein Aminoisoindolenin der Formel (V) 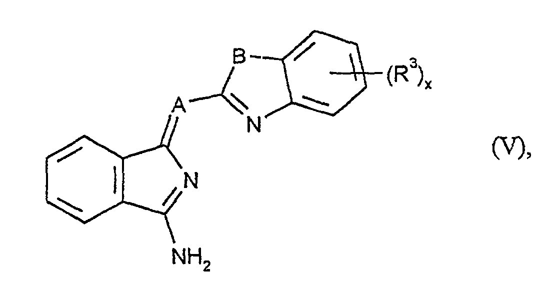 mit einem Cyanessigsäureamid der Formel (VI) 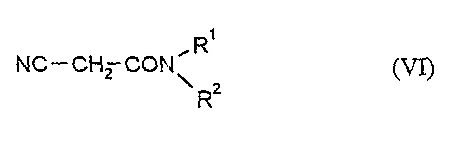 kondensiert, wobei
- R¹, R², R³ A, B und x: die oben angegebene Bedeutung besitzen.

Die Verbindungen der Formel (III) können beispielsweise durch die Formeln (Va) sowie (Vb) oder ihre tautomeren Formeln individualisiert werden.

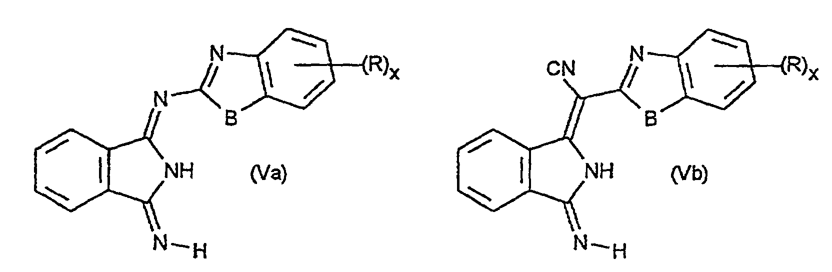

Verbindungen der Formel (III) können in ähnlicher Weise wie die in DE-A-1 670 748 oder in DE-A-2 041 999 beschriebenen analogen Verbindungen hergestellt werden.

Besonders bevorzugte Verbindungen der Formel (III) sind solche, bei denen R² verschieden von Wasserstoff ist.

Bei einem Verfahren zu ihrer Herstellung setzt man Amino-imino-isoindolin mit einem Cyanessigsäureamid der Formel (VI) um, wobei man bevorzugt mit einem polaren, insbesondere hydrophilen organischen Lösungsmittel arbeitet. Auch Wasser und Mischungen von Wasser mit Lösungsmitteln sind geeignet. Als polare Lösungsmittel sind beispielsweise zu nennen: Amide wie Dimethylformamid, Formamid, Dimethylacetamid, N-Methylpyrrolidon, ferner Dimethylsulfoxid, Acetonitril, Essigsäure oder bevorzugt Alkohole, wie z.B. Methanol, Ethanol, Propanol, Isopropanol, Butanol, Methylglykol oder Ethylglykol. Darüber hinaus können auch Mischungen dieser Lösungsmittel Verwendung finden.

Die erforderlichen Reaktionstemperaturen, liegen zwischen ca. 0 und 100°C, bevorzugt zwischen 20 und 80°C.

Bei einem anderen Verfahren zur Herstellung von Verbindungen der Formel (III) lagert man zunächst in Gegenwart eines Alkoholates als Katalysator einen Alkohol an Phthalodinitril an zu einer Zwischenverbindung (siehe I. Chembrier u. M.J. Cook, J. Chem. Research, 322 (1990) und F. Baumann et al., Angew. Chem., 68, 133 (1956) sowie EP 510 436). Die Zwischenverbindung kann ohne vorangehende Isolierung weiter mit einem Cyanessigsäureamid der Formel (VI) umgesetzt werden, wobei man zur Beschleunigung der Reaktion organische Säuren zusetzen kann. Geeignete Alkohole sind insbesondere niedere Alkohole wie z.B. Methanol, Ethanol, Propanol und Isopropanol. Die erforderlichen Reaktionstemperaturen liegen bevorzugt zwischen 20 und 80°C.

Die Verbindungen der Formel (III) können zwischenisoliert werden. Man kann sie aber auch aus dem Reaktionsansatz, gegebenenfalls nach Zusatz von weiterem Lösungsmittel und/oder Wasser unmittelbar weiterkondensieren.

Die Verbindungen der Formel (III), worin
- R¹: für CH₃ steht, und
- R²: für -CH₂CH₂OH oder -CH₂CH₂CH₂CH₃ steht,
oder
- R¹ und R²: gemeinsam mit dem N- Atom, an das sie gebunden sind, für einen Rest 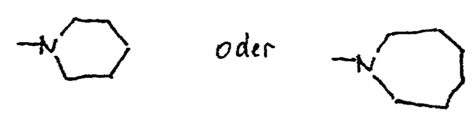 stehen, sind neu und ebenfalls Gegenstand der vorliegenden Erfindung.

Verbindungen der Formeln (Va) und (Vb) werden ebenfalls in DE-A-1 670 748 beschrieben.

In einer bevorzugten Ausführungsform des erfindungsgemäßen Verfahrens zur Herstellung von Verbindungen der Formel (I) erfolgt die Kondensation in Wasser, in einem polaren, insbesondere hydrophilen organischen Lösungsmittel oder Mischungen davon.

Als polare Lösungsmittel sind beispielsweise zu nennen: Amide wie Dimethylformamid, Formamid Dimethylacetamid, N-Methylpyrrolidon, ferner Dimethylsulfoxid, Acetonitril, Essigsäure oder Alkohole, wie z.B. Methanol, Ethanol, Butanol, Methylglykol oder Ethylglykol. Darüber hinaus können auch Mischungen dieser Lösungsmittel Verwendung finden.

Besonders bevorzugt erfolgt das erfindungsgemäße Verfahren in Gegenwart einer organischen Säure. Diese führt zu einer Beschleunigung der Reaktion, wobei oft auch höhere Ausbeuten und eine höhere Reinheit erzielt werden. Geeignete organische Säuren sind z.B. niedere aliphatische, gesättigte oder ungesättigte Mono- oder Dicarbonsäuren wie z.B. Ameisensäure, Essigsäure, Propionsäure, Buttersäure, Oxalsäure, Fumarsäure, Maleinsäure, Bernsteinsäure, Glutarsäure, Adipinsäure, aber auch aromatische Säuren wie beispielsweise Benzoesäure, Phthalsäure, Phenylessigsäure, Iso- und Terephthalsäure. Die Säuren werden in Mengen von 0,2 - 3 Mol-Äquivalenten, vorzugsweise 1 - 2 Mol-Äquivalenten zugegeben. Aber auch höhere Mengen an Säure können eingesetzt werden, bevorzugt dann, wenn die Säure gleichzeitig als Lösungsmittel dient, beispielsweise Essigsäure.

Die Komponenten können in äquivalenten Mengen oder im Überschuß eingesetzt werden.

Die Reaktionstemperaturen für das erfindungsgemäße Verfahren liegen vorzugsweise bei 10 bis 140°C, insbesondere bei 20 bis 120°C und können je nach der Reaktionsfähigkeit der Ausgangsprodukte variiert werden.

Vorzugsweise erfolgt die Umsetzung von Verbindungen der Formel (III) mit Verbindungen der Formel (IVa) bei einer Temperatur von 90 bis 140°C, und wird bevorzugt in einem organischen Lösungsmittel durchgeführt, das bei der Reaktionstemperatur oder darüber siedet.

Vorzugsweise erfolgt die Umsetzung von Verbindungen der Formel (III) mit Verbindungen der Formel (IVb) und von Verbindungen der Formel (Va) oder (Vb) mit Verbindungen der Formel (VI) bei einer Temperatur von 10 bis 100°C, wobei die Kondensation vorzugsweise in Wasser oder in einer Mischung aus Wasser und einem organischen Lösungsmittel durchgeführt werden kann.

Das Arbeiten mit Wasser bzw. wasserhaltigen Reaktionsmedien erleichtert die Isolierung der Farbstoffe und vermeidet die Aufarbeitung von größeren Mengen an organischen Lösungsmitteln. Bevorzugt wird bei dieser Verfahrensvariante mit Wassergehalten von 20 bis 100 %, insbesondere 50 bis 100 % gearbeitet, bezogen auf die Menge des verwendeten Reaktionsmediums.

Verwendet man Wasser oder ein überwiegend wäßriges Medium als Reaktionsmedium, so werden zweckmäßigerweise oberflächenaktive Substanzen wie Tenside, Dispergiermittel, Emulgatoren und Netzmittel zugegeben. In Betracht kommen die bekannten nichtionogenen, anionischen und kationischen Hilfsmittel. Solche Verbindungen sind z.B. Salze von Alkylbenzolsulfonsäuren, Alkylphenolsulfonsäuren, Alkylnaphthalinsulfonsäuren, Kondensationsprodukte aus Phenolsulfonsäuren, Fomaldehyd und Harnstoff, Ligninsulfonate, Additionsprodukte von Ethylen- und Propylenoxid an Alkanole, Alkandiole, Phenole, Carbonsäuren, Amine, Carbonsäureamide und ihre Schwefelsäurehalbester, wobei auch Mischungen dieser Verbindungen eingesetzt werden können. Besonders bevorzugt sind jedoch Ligninsulfonate wie z.B. Kraftlignine vom Typ Reax der Firma Westvaco oder Sulfitlignine vom Typ Ufoxane der Firma Borregaard.

Die Erfindung betrifft weiterhin die Verwendung von Verbindungen der Formel (I) zum Färben von vollsynthetischen oder halbsynthetischen, hochmolekularen Stoffen. Besonders geeignet sind sie zum Färben oder Bedrucken von synthetischen Fasermaterialien, insbesondere solchen aus aromatischen Polyestern und/oder Celluloseacetaten. Die dabei erhaltenen Färbungen besitzen eine hohe Farbstärke und zeigen eine überragende Lichtechtheit, insbesondere eine hohe Heißlichtechtheit, und sind daher besonders zum Färben und Bedrucken von Textilmaterialien für Automobile sowie für das Färben von sogenannten Microfasern geeignet. Sie eignen sich auch für das sogenannte Thermo-Transfer-Printing auf textilen und nichttextilen Substraten z.B. nach dem D2T2 (Dye Diffusion Thermo Transfer) - Prozess für die Bildaufzeichnung. Weiterhin können die Farbstoffe zum Massefärben von Kunststoffen z.B. von Polyethylen, Polypropylen, Styrol, Polycarbonaten sowie von Kunststoffblends wie z.B. ABS verwendet werden. Die Farbstoffe, insbesondere diejenigen, bei denen A für einem Cyanmethylenrest steht, fluoreszieren teilweise und eignen sich daher auch als Fluoreszenzfarbstoffe.

Textilmaterialien aus Polyester können mit den erfindungsgemäßen Farbstoffen nach Art einer Spinnfärbung gefärbt werden, bevorzugt werden sie jedoch aus wäßriger Suspension gefärbt. Hierzu werden die Farbstoffe auf allgemein bekannte Weise zu Färbepräparaten verarbeitet, z.B. durch Mahlen in Wasser in Gegenwart von Dispergier- und/oder Füllmitteln. Mit den gegebenenfalls im Vakuum oder durch Zerstäuben getrockneten Präparaten kann man nach Zugabe von Wasser in sogenannter kurzer oder langer Flotte färben, klotzen oder bedrucken.

Zur Herstellung bzw. Verbesserung des Dispersionsgrades kann bei der Mahlung oder der Synthesereaktion ein oberflächenaktives Mittel oder ein Gemisch derartiger Hilfsmittel zugesetzt werden. Selbstverständlich kann die Teilchengröße der Farbstoffpartikel durch eine Mahlbehandlung z.B. Naßperlmahlung, sei es während der Synthese oder im Anschluß daran entsprechend beeinflußt und auf einen geforderten Wert eingestellt werden.

Als Dispergiermittel kommen solche anionischer oder nicht anionischer Natur in Betracht. Neben Dispergiermitteln der einen oder anderen Gruppe können auch Dispergiermittelgemische eingesetzt werden, wobei in erster Linie Gemische von nichtionischen und anionischen Dispergiermitteln gemeint sind, da anionische und kationische Dispergiermittel im Gemisch untereinander zur Bildung von Ausfällungen neigen.

Bei den anionschen Dispergiermitteln haben sich insbesondere Kondensationsprodukte von aromatischen Sulfonsäuren mit Formaldehyd, wie Kondensationsprodukte aus Formaldehyd und Alkylnaphthalinsulfonsäuren oder aus Formaldehyd, Naphthalinsulfonsäuren und Benzolsulfonsäure, Kondensationsprodukte aus gegebenenfalls substituiertem Phenol mit Formaldehyd und Natriumbisulfit als wirksam erwiesen.

Weiterhin kommen vor allem Ligninsulfonate in Betracht, z.B. solche, die nach dem Sulfit- oder Kraft-Verfahren gewonnen werden. Vorzugsweise handelt es sich um Produkte, die z.T. hydrolysiert, oxidiert, propoxyliert oder desulfoniert und nach bekannten Verfahren fraktioniert werden, z.B. nach dem Molekulargewicht oder nach dem Sulfonierungsgrad. Auch Mischungen aus Sulfit- und Kraftligninsulfonaten sind gut wirksam.

Besonders geeignet sind Ligninsulfonate mit einem durchschnittlichen Molekulargewicht zwischen 1.000 und 100.000, einem Gehalt an aktivem Ligninsulfonat von mindestens 80% und vorzugsweise mit niedrigem Gehalt an mehrwertigen Kationen.

Der Sulfonierungsgrad kann in weiten Grenzen variieren.

Nichtionische Dispergiermittel oder Emulgatoren sind z.B. Umsetzungsprodukte von Alkylenoxiden mit alkylierbaren Verbindungen, wie z.B. Fettalkoholen, Fettaminen, Fettsäuren, Phenolen, Alkylphenolen, Arylalkylphenolen und Carbonsäureamiden.

Hierbei handelt es sich z.B. um Ethylenoxid-Addukte aus der Klasse der Umsetzungsprodukte von Ethylenoxid mit:
a) gesättigten und/oder ungesättigten Fettalkoholen mit 6 bis 20 C-Atomen; oder
b) Alkylphenolen mit 4 bis 12 C-Atomen im Alkylrest, oder
c) gesättigten und/oder ungesättigten Fettaminen mit 14 bis 20 C-Atomen, oder
d) gesättigten und/oder ungesättigten Fettsäuren mit 14 bis 20 C-Atomen,
e) hydrierte und/oder unhydrierte Harzsäuren.

Als Ethylenoxid-Addukte sind im einzelnen genannt:
a) Umsetzungsprodukte von gesättigten und/oder ungesättigten Fettalkoholen mit 6 bis 20 C-Atomen, mit 5 bis 30 Mol Ethylenoxid
b) Umsetzungsprodukte von Alkylphenolen mit 4 bis 12 C-Atomen mit 5 bis 20 Mol Ethylenoxid
c) Umsetzungsprodukte von gesättigten und/oder ungesättigten Fettaminen mit 14 bis 20 C-Atomen mit 5 bis 20 Mol Ethylenoxid
d) Umsetzungsprodukte von gesättigten und/oder ungesättigten Fettsäuren mit 14 bis 20 C-Atomen mit 5 bis 20 Mol Ethylenoxid.

Weitere bevorzugte Dispergiermittel sind alkoxylierte Styrol-Phenol-Kondesationsprodukte, die gegebenenfalls im Gemisch mit ihren anorganichen Estern, die durch Umsetzung des alkoxylierten Styrol-Phenol-Kondesationsproduktes mit anorganischen Säuren, wie beispielsweise Amidosulfonsäure, erhalten werden, eingesetzt werden.

Insbesondere zum Färben von Polyester eigenen sich auch Mischungen von Farbstoffen der Formeln (I), wodurch unter Umständen das Zieh- und Aufbauvermögen der Farbstoffe sowie ihre Dispergierbarkeit verbessert werden können.

Die neuen Farbstoffmischungen können nach verschiedenen Verfahren hergestellt werden:
1. durch Abmischen der separat hergestellten und formierten Einzelfarbstoffkomponenten,
2. durch gemeinsame Formierung der separat hergestellten Einzelkomponenten,
3. z.B. durch gemeinsame Synthese von Mischungen der Farbstoffe der Formeln (I), worin x für 0 oder 1 steht und Farbstoffe der Formel (II) aus Mischungen unterschiedlicher Vorprodukte.

Die Mischung der Farbstoffe erfolgt zweckmäßigerweise in geeigneten Mühlen, beispielsweise Kugel- oder Sandmühlen. Getrennt formierte Einzelfarbstoffe können aber auch durch Einrühren in Färbeflotten gemischt werden.

Besonders geigenet sind Mischungen von Farbstoffe der Formel (I), die sich lediglich in dem Rest R₁ und R₂ unterscheiden.

Die Farbstoffe eignen sich jedoch auch hervorragend zur Herstellung von Mischungen mit anderen Dispersionsfarbstoffen zur Erzeugung z.B. von Braun-, Grau- oder Grüntönen auf der Faser.

Eine weitere bevorzugte Ausführungsform der vorliegenden Erfindung betrifft Mischungen von einem oder mehreren der Farbstoffe der Formel (I) mit einem oder mehreren Farbstoffen, wie sie üblicherweise zum Färben von Polyesterfasern oder Polyestertextilmaterialien für Automobilbezugsstoffe verwendet werden. Bei diesen Farbstoffen zum Färben von Automobilbezugsstoffen kann es sich insbesondere um Azo- Disazo-, Anthrachinon-, Nitro-, Naphthalimid- und Terephthalimid-Farbstoffe handeln. Besonders bevorzugte Farbstoffe für derartige Mischungen sind z.B. die Colour-Index-Farbstoffe Yellow 23, 42, 51, 59, 65, 71, 86, 108, 122, 163, 182, 211, Orange 29, 30, 32, 41, 44, 45, 61, 73, Rot 60, 82, 86, 91, 92, 127, 134, 138, 159, 167, 191, 202, 258, 279, 284, 302, 323, Blau 27, 54, 56, 60, 73, 77, 79, 79:1, 87, 266, 333, 361 Violet 27, 28, 57, und 95, wobei sich die Gewichtsverhältnisse der Farbstoffmischungen nach der gewünschten Farbnuance richten.

### Beispiele

### Beispiel 1:

234,7 g (1,5 Mol) technisches Amino-imino-isoindolenin (92,8 %-ig) und 228,2 g Cyanessigsäure-piperidid der Formel (VI), wobei R₁ und R₂ gemeinsam einen Pentamethylenrest bilden, wurden mit 800 ml Methanol verrührt und 6 Stunden bei Raumtemperatur und 5 Stunden unter Rückfluß gehalten. Die ausgefallene Substanz wurde bei Raumtemperatur abgesaugt und mit Methanol und Wasser gewaschen. Nach dem Trocknen erhielt man 199,8g (87,5% d.Th.) eines Produktes der Formel:

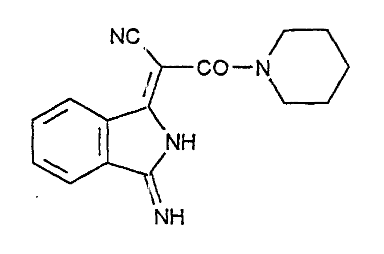

### Beispiel 2:

28 g (0,1 Mol) des Produktes aus Beispiel 1, 17,4 g (0,1 Mol) 2-Cyanmethylbenzthiazol und 6 ml Eisessig wurden mit 100 ml N-Methyl-pyrrolidon über Nacht bei Raumtemperatur verrührt. Danach wurden 50 ml Methanol zugegeben und der Ansatz noch 3 Stunden unter Rückfluß gehalten. Das ausgefallenen Produkt wurde bei Raumtemperatur abgesaugt und mit Wasser und Methanol gewaschen. Man erhielt 29,8 g eines Farbstoffes der Formel:

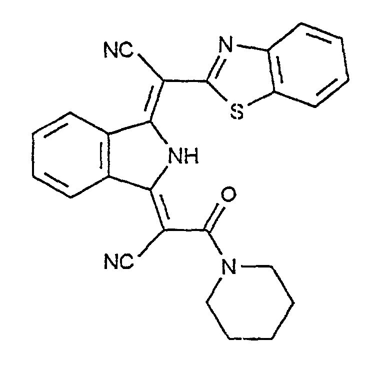

Der Farbstoff färbt Polyesterfasern in fluoreszierenden gelben Tönen mit hervorragender Lichtechtheit

### Beispiel 3:

132 ml (1,5 Mol) Cyanessigsäuremethylester und 182 ml (1,575 Mol) Methyl-butylamin wurden 8 Stunden unter Rückfluß erhitzt und danach 400 ml Methanol und 234,7 g (1,5 Mol) technisches Amino-imino-isoindolenin (92,8 %-ig) zugegeben. Der Ansatz wird 1 Stunde bei Raumtemperatur und danach noch 5 Stunden unter Rückfluß gehalten. Nach Abkühlen in einem Eisbad wurde die ausgefallene Substanz abgesaugt und mit eiskaltem Methanol und mit Wasser gewaschen. Nach dem Trocknen erhielt man 325 g (76,9% d.Th.) eines Produktes der folgenden Formel.

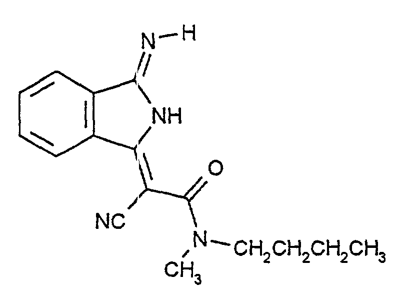

### Beispiel 4:

28,2 g (0,1 Mol) des Produktes aus Beispiel 3, 17,4 g (0,1 Mol) 2-Cyanmethylbenzthiazol 100 ml Eisessig und 50 ml Wasser wurden 2 Stunden bei Raumtemperatur und noch 4 Stunden bei 50° verrührt. Das ausgefallenen Produkt wurde bei Raumtemperatur abgesaugt und mit Wasser und Methanol gewaschen. Man erhielt 32,5 g eines Farbstoffes der Formel:

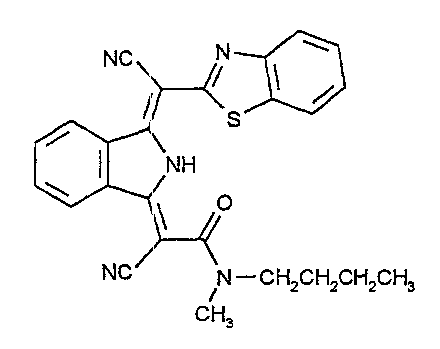

Der Farbstoff färbt Polyesterfasern in fluoreszierenden gelben Tönen mit ebenfalls hervorragender Lichtechtheit.

### Beispiel 5:

Verfuhr man wie bei Beispiel 4, setzte jedoch an Stelle von 2-Cyanmethylbenzthiazol 2-Cyanmethyl-benzimidazol ein, so erhielt man in 34,3 g des Farbstoffes der Formel:

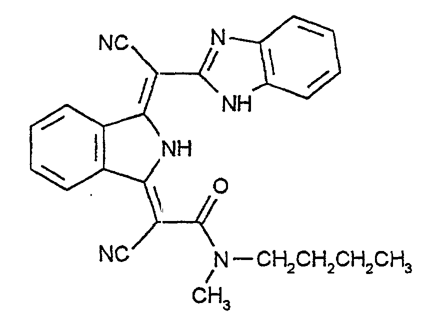

Der Farbstoff färbt Polyesterfasern ebenfalls in gelben Tönen mit hervorragender Lichtechtheit

### Beispiel 6:

28,2 g (0,1 Mol) der Substanz von Beispiel 3, 17 g (0,11 Mol) 2-Aminobenzthiazol, 6 ml Eisessig und 150 ml n-Butanol wurden 7 Stunden unter Rückfluß erhitzt. Nach Abkühlen auf Raumtemperatur wurden der ausgefallene Farbstoff abgesaugt und mit Methanol und Wasser gewaschen. Man erhielt 26,7 g eines Farbstoffes der folgenden Formel, der Polyesterfasern grünstichig gelb anfärbt, wobei die Färbungen ebenfalls hervorragende Echtheiten aufweisen.

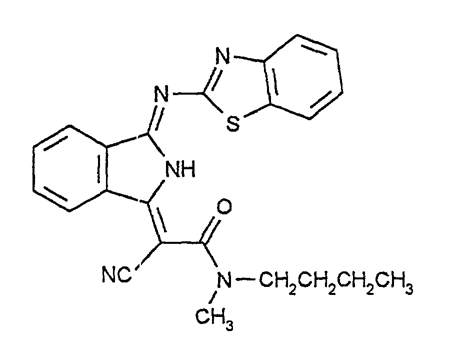

### Beispiel 7:

Verfuhr man analog Beispiel 3, verwendete aber statt Methyl-butylamin (2-Hydroxyethyl)-methylamin, so erhielt man in vergleichbarer Ausbeute ein Produkt der Formel:

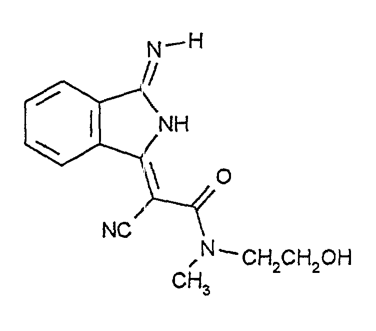

### Beispiel 8:

27 g (0,1 Mol) des Produktes aus Beispiel 7 wurden analog zu Beispiel 6 mit 2-Amino-benzthiazol umgesetzt und isoliert. Die getrocknete Substanz wurde mit 150 ml Eisessig und 12 ml Acetanhydrid 5 Stunden auf 80° erhitzt. Nach Abkühlen auf Raumtemperatur wurde die Fällung durch Zugabe von etwas Wasser vervollständigt, abgesaugt und mit Wasser gewaschen. Man erhielt 19,3 g eines Farbstoffes der Formel:

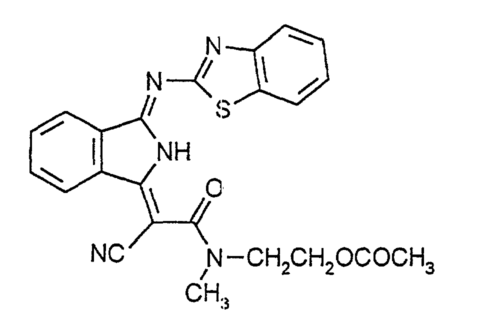

Der Farbstoff zeigt analoge Eigenschaften wie der Farbstoff von Beispiel 6.

### Beispiel 9:

159,7 g (1,1 Mol) Amino-imino-isoindolenin, 150,2 g (1,0 Mol) 2-Aminobenzthiazol und 750 ml Ethanol wurden 24 Stunden unter Rückfluß erhitzt. Nach Abkühlen auf Raumtemperatur wurde das ausgefallene Produkt abgesaugt und mit Ethanol und Wasser gewaschen. Nach dem Trocknen bei 70° erhielt man 226,9 g (81,5% d.Th.) eines Produktes der Formel:

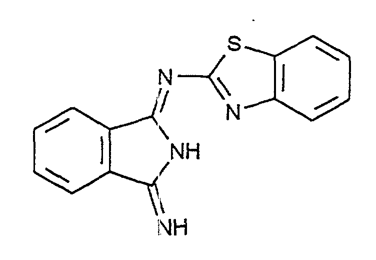

### Beispiel 10:

27,8 g (0,1 Mol) der in Beispiel 9 hergestellten Verbindung und 16,7 g (0,1 Mol) des Umsetzungsproduktes von Cyanessigsäuremethylester mit Hexamethylenimin wurden mit 150 ml Methylglykol verrührt und 10 Stunden auf 110° erhitzt. Nach der Isolierung erhielt man 30,3 g des Farbstoffes der folgenden Formel, der Polyesterfasern in ähnlichen Tönen mit vergleichbaren Echtheiten färbt wie der Farbstoff in Beispiel 5.

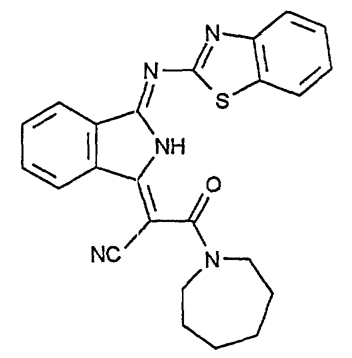

### Beispiel 11:

Verfuhr man wie in Beispiel 1, setzte jedoch statt des Cyanessigsäure-piperidids das analoge Umsetzungsprodukt von Cyanessigsäuremethylester mit Hexamethylenimin ein, so erhielt man in vergleichbarer Ausbeute das Produkt der Formel:

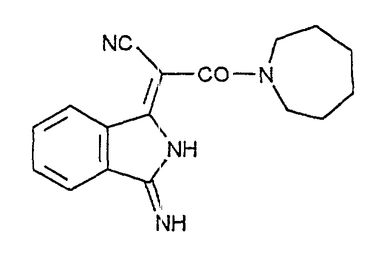

### Beispiel 12:

29,4 g (0,1 Mol) der in Beispiel 11 hergestellten Verbindung, 17,4 g 2-Cyanmethyl-benzthiazol und 150 ml Eisessig wurden 2 Stunden bei Raumtemperatur und 4 Stunden bei 50° verrührt. Das Produkt wurde bei Raumtemperatur abgesaugt und mit Methanol und Wasser gewaschen. Man isolierte so 34,7 g eines Farbstoffes der Formel:

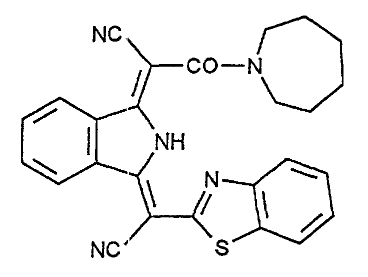

Der Farbstoff färbt Polyesterfasern in gelben fluoreszierenden Tönen.

In analoger Weise wurden die Farbstoffe der nachfolgenden Tabellenbeispiele hergestellt, wobei die Substituenten der Verbindung VII die in der Tabelle angegebene Bedeutung besitzt.

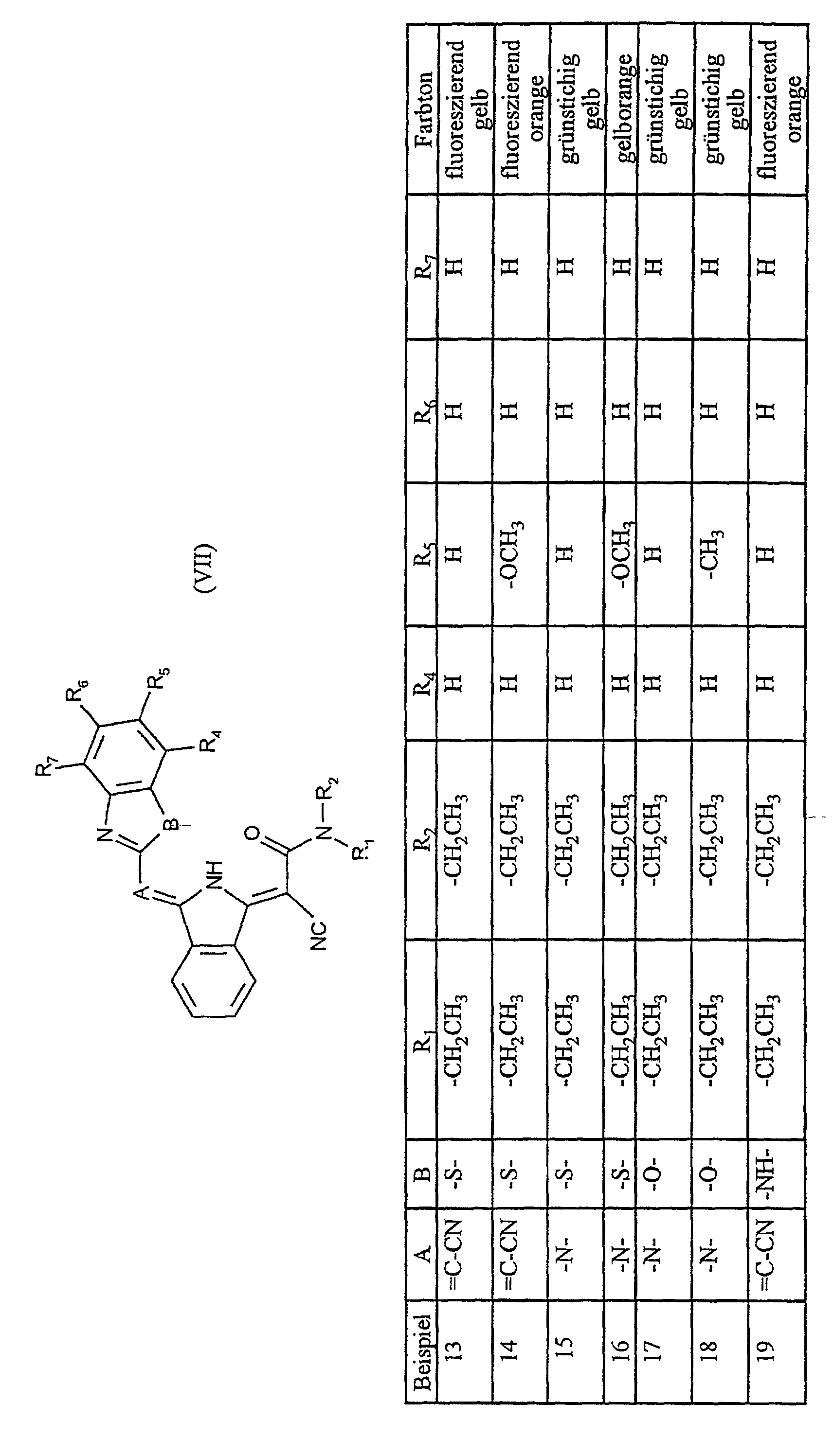

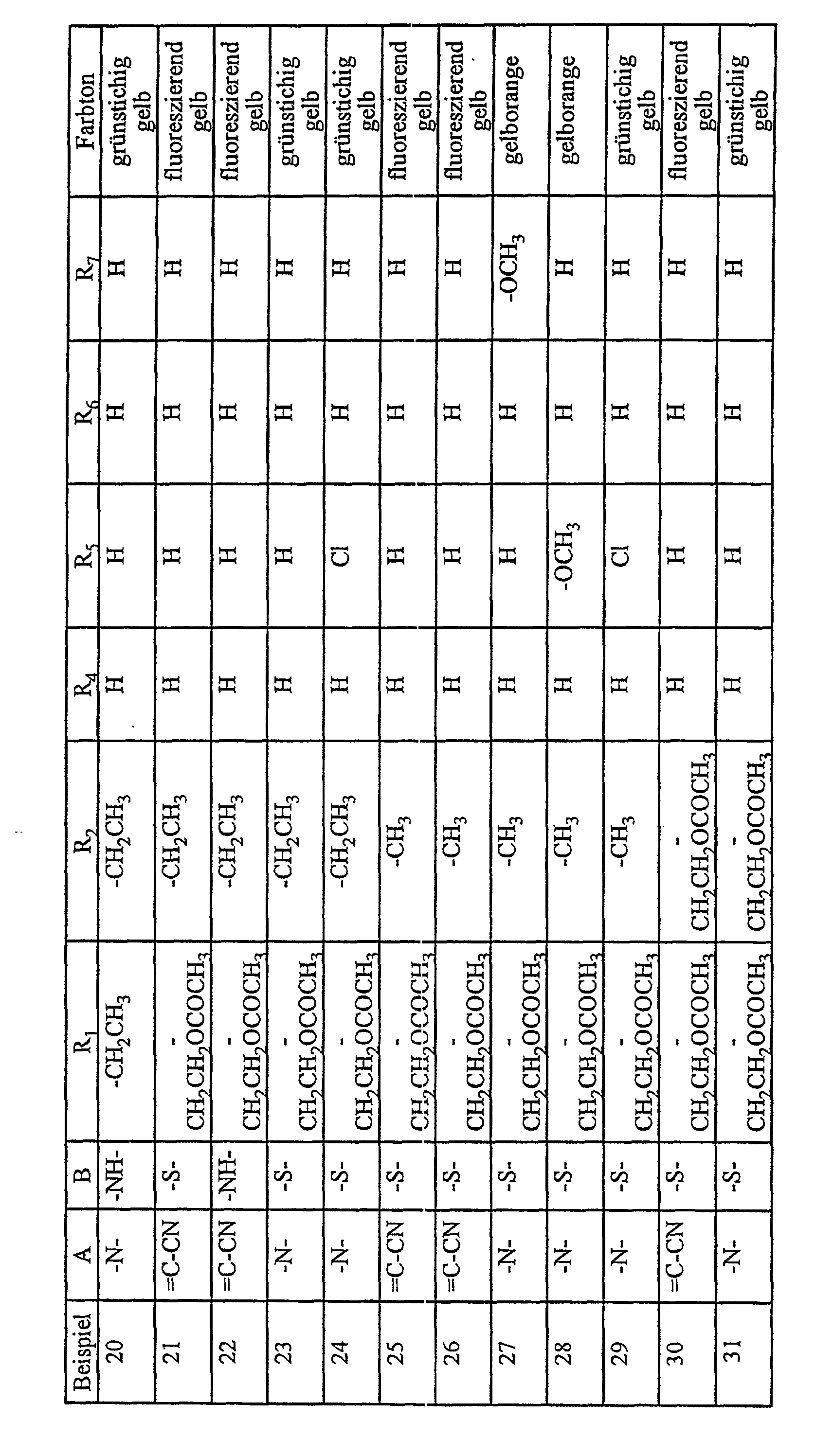

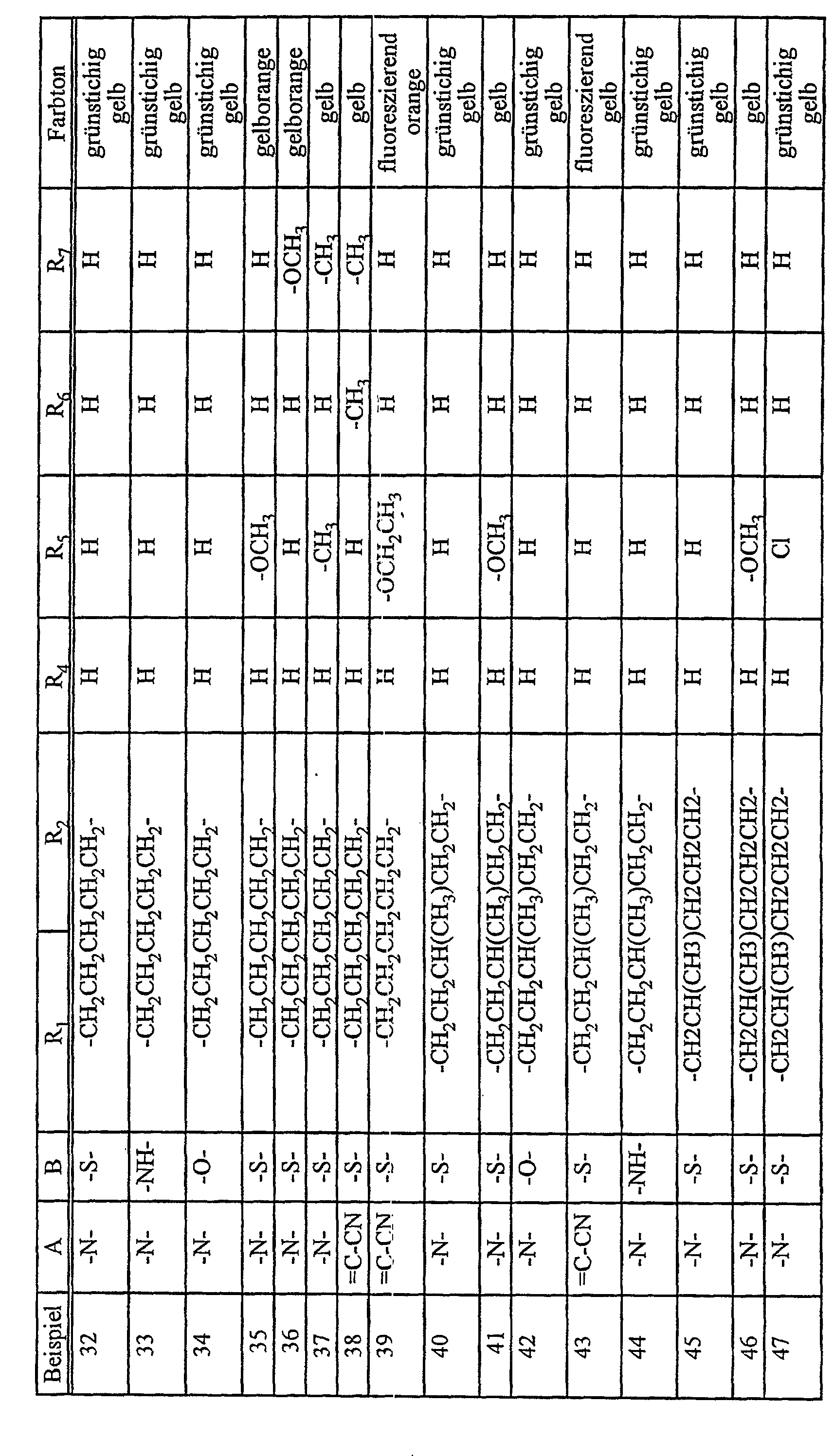

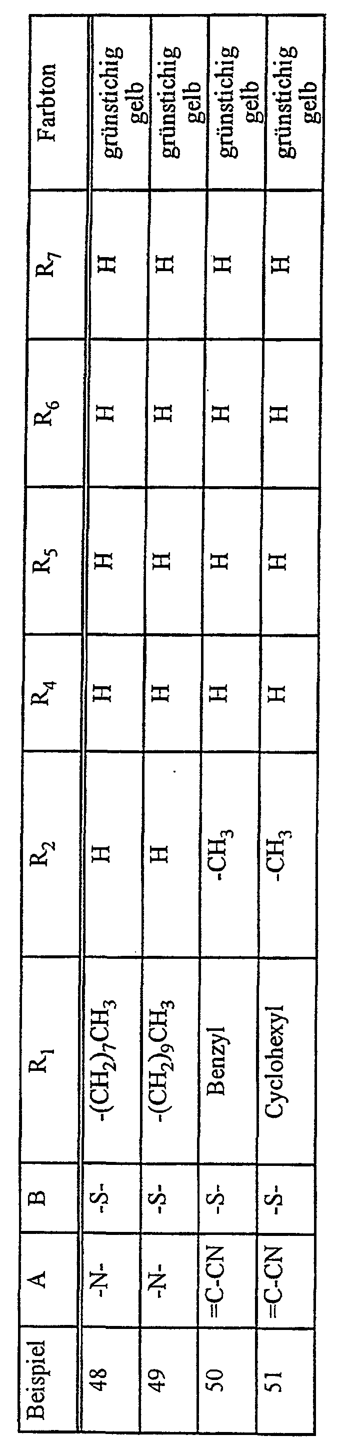

## Patentansprüche

1. Verbindungen der Formel (I) 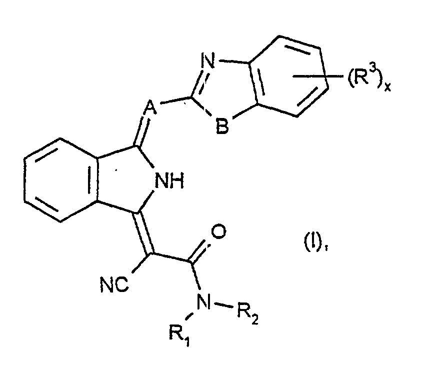 worin
A für N oder einen Cyanmethylenrest steht,
B für S, O oder NH steht,
R¹ für einen gesättigten oder ungesättigten, aliphatischen Rest mit 1 bis 12 C-Atomen, insbesondere 1 bis 10 C-Atomen, steht, der gegebenenfalls durch ein oder mehrere Sauerstoffatome unterbrochen ist und/oder gegebenenfalls durch einen oder mehrere gleich oder verschiedene Substituenten aus der Reihe Alkoxy, Acyloxy, Halogen, CN, Aryl, insbesondere Phenyl substituiert ist,
R² Wasserstoff oder Aryl bedeutet oder eine Bedeutung annimmt, wie sie für R¹ angegeben ist, wobei R¹ und R² gleich oder verschieden sind oder
R¹ und R² gemeinsam mit dem N-Atom, an das sie gebunden sind, einen heterocyclischen Ring bilden,
R³ Halogen, insbesondere Cl, F und Br, C₁-C₄-Alkyl, ein gesättigter oder ungesättigter aliphatischer Oxyrest mit 1 bis 4 C-Atomen, insbesondere C₁-C₄-Alkoxy, der gegebenenfalls durch C₁-C₄Alkoxy substituiert ist, CN oder NO₂ bedeutet, und
x eine Zahl von 0 bis 4 bedeutet.

2. Verbindungen gemäß Anspruch 1, die der Formel (I) oder deren tautomeren Formen entsprechen
worin
x für 0 oder 1 steht.

3. Verbindungen gemäß Anspruch 1, die der Formel (II) oder deren tautomeren Formen entsprechen 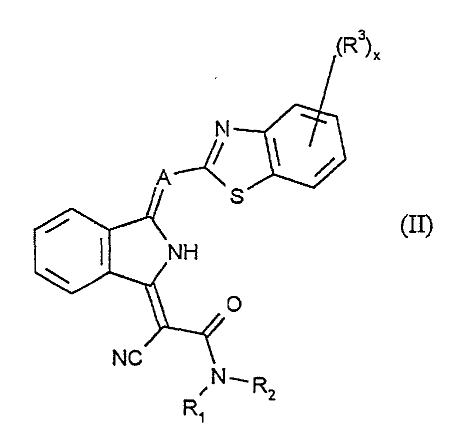 worin A, R¹ - R³ und x die in Anspruch 1 angegebene Bedeutung besitzen.

4. Verbindungen gemäß Anspruch 1, die der Formel (I) oder deren tautomeren Formen entsprechen, worin R³ für Chlor, Methyl, Methoxy oder Ethoxy steht.

5. Verfahren zur Herstellung von Verbindungen gemäß Anspruch 1, **dadurch gekennzeichnet**, daß man eine Verbindung der Formel (III) 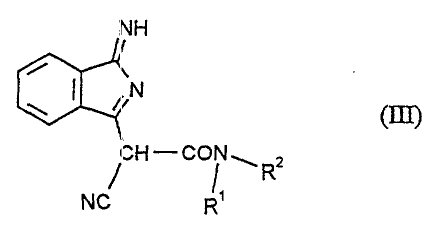 mit einer Verbindung der Formel (IV) 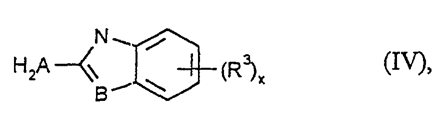 oder ein Aminoisoindolenin der Formel (V) 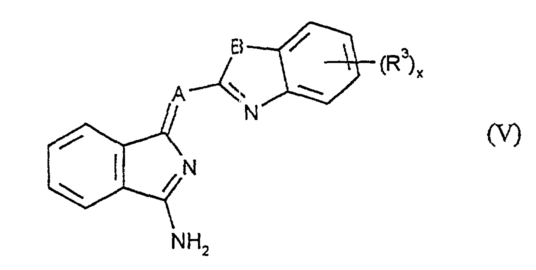 mit einem Cyanessigsäureamid der Formel (VI) 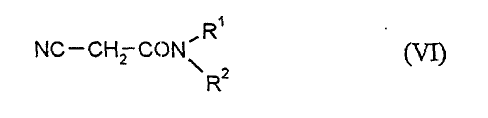 zu Verbindungen der Formel (I) kondensiert 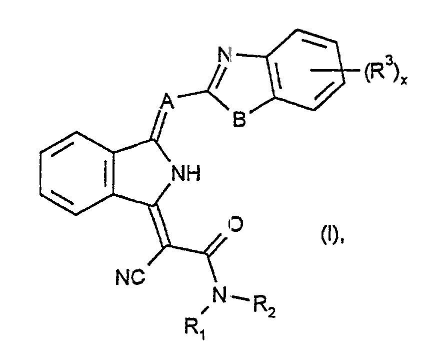 worin
A für N oder einen Cyanmethylenrest steht,
B für S, O oder NH steht,
R¹ für einen gesättigten oder ungesättigten, aliphatischen Rest mit 1 bis 12 C-Atomen, insbesondere 1 bis 10 C-Atomen, steht, der gegebenenfalls durch ein oder mehrere Sauerstoffatome unterbrochen ist und/oder gegebenenfalls durch einen oder mehrere gleiche oder verschiedene Substituenten aus der Reihe Alkoxy, Acyloxy, Halogen, CN, Aryl, insbesondere Phenyl substituiert ist,
R² Wasserstoff oder Aryl bedeutet oder eine Bedeutung annimmt, wie sie für R¹ angegeben ist, wobei R¹ und R² gleich oder verschieden sind, oder
R¹ und R² gemeinsam mit dem N-Atom, an das sie gebunden sind, einen heterocyclischen Ring bilden,
R³ Halogen, insbesondere Cl, F und Br, C₁-C₄-Alkyl, ein gesättigter oder ungesättigter aliphatischer Oxyrest mit 1 bis 4 C-Atomen, insbesondere C₁-C₄-Alkoxy, der gegebenenfalls durch C₁-C₄Alkoxy substituiert ist, CN oder NO₂ bedeutet,
und
x eine Zahl von 0 bis 4 bedeutet

6. Verwendung von Verbindungen gemäß Anspruch 1 zum Färben oder Bedrucken von voll- oder halbsynthetischen hochmolekularen Stoffen.

7. Verwendung gemäß Anspruch 5, **dadurch gekennzeichnet**, daß Automobilbezugsstoffe gefärbt oder bedruckt werden.

8. Verbindungen der Formel (III) 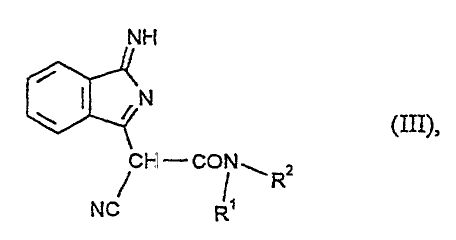 worin
R¹ für CH₃ steht, und
R² für -CH₂CH₂OH oder -CH₂CH₂CH₂CH₃ steht,
oder
R¹ und R² gemeinsam mit dem N-Atom, an das sie gebunden sind, für einen Rest 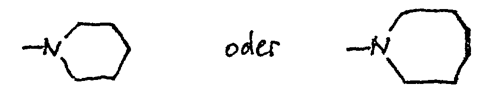 stehen.

## Claims

1. Compounds of the formula (I) 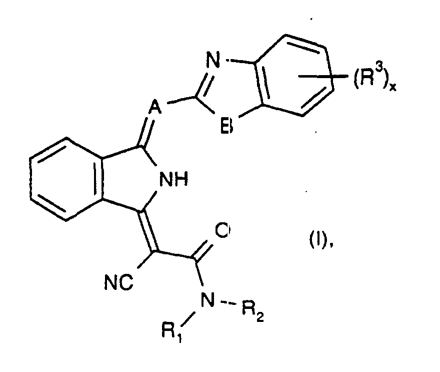 in which
A represents N or a cyanomethylene radical,
B represents S, O or NH,
R¹ represents a saturated or unsaturated aliphatic radical having 1 to 12 C atoms, in particular 1 to 10 C atoms, which is uninterrupted or interrupted by one or more oxygen atoms and/or is unsubstituted or substituted by one or more identical or different substituents selected from the group consisting of alkoxy, acyloxy, halogen, CN, aryl, in particular phenyl,
R² denotes hydrogen or aryl or adopts one of the meanings given for R¹, R¹ and R² being identical or different,
or
R¹ and R², together with the N atom to which they are bonded, form a heterocyclic ring,
R³ denotes halogen, in particular Cl, F and Br, C₁-C₄-alkyl, a saturated or unsaturated aliphatic alkoxy radical having 1 to 4 C atoms, in particular C₁-C₄-alkoxy, which is unsubstituted or substituted by C₁-C₄-alkoxy, CN or NO₂,
and
x denotes a number from 0 to 4.

2. Compounds according to Claim 1, having the formula (I) or corresponding to tautomeric forms thereof
in which
x represents 0 or 1.

3. Compounds according to Claim 1, having the formula (II) or corresponding to tautomeric forms thereof 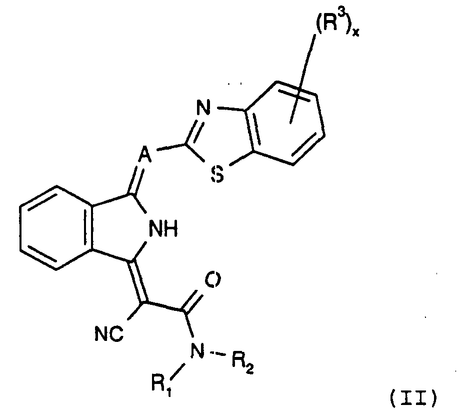 in which A, R¹ - R³ and x have the meaning given in Claim 1.

4. Compounds according to Claim 1, having the formula (I) or corresponding to tautomeric forms thereof, in which R³ represents chlorine, methyl, methoxy or ethoxy.

5. Process for preparing compounds according to Claim 1, **characterized in that** a compound of the formula (III) 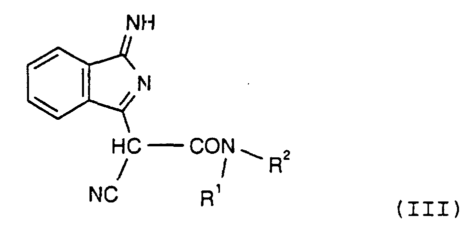 is condensed with a compound of the formula (IV) 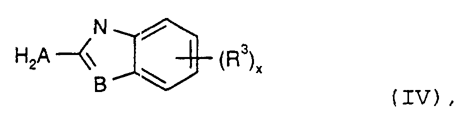 or an aminoisoindolenine of the formula (V) 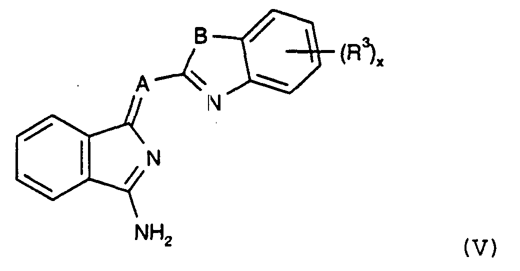 is condensed with a cyanoacetamide of the formula (VI) 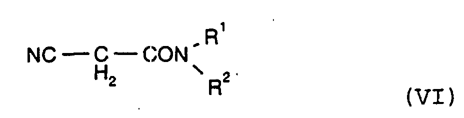 to give compounds of the formula (I) 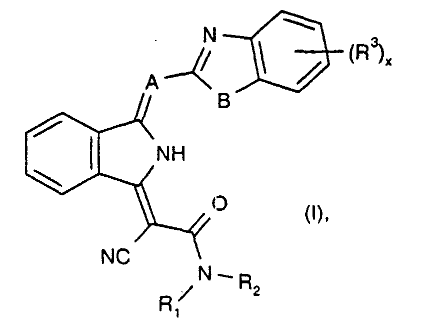 in which
A represents N or a cyanomethylene radical,
B represents S, O or NH,
R¹ represents a saturated or unsaturated aliphatic radical having 1 to 12 C atoms, in particular 1 to 10 C atoms, which is uninterrupted or interrupted by one or more oxygen atoms and/or is unsubstituted or substituted by one or more identical or different substituents selected from the group consisting of alkoxy, acyloxy, halogen, CN, aryl, in particular phenyl,
R² denotes hydrogen or aryl or adopts one of the meanings given for R¹, R¹ and R² being identical or different,
or
R¹ and R², together with the N atom to which they are bonded, form a heterocyclic ring,
R³ denotes halogen, in particular Cl, F and Br, C₁-C₄-alkyl, a saturated or unsaturated aliphatic alkoxy radical having 1 to 4 C atoms, in particular C₁-C₄-alkoxy, which is unsubstituted or substituted by C₁-C₄-alkoxy, CN or NO₂,
and
x denotes a number from 0 to 4.

6. Use of compounds according to Claim 1 for dyeing or printing fully synthetic or semisynthetic high-molecular-weight materials.

7. Use according to Claim 5, **characterized in that** automotive cover fabrics are dyed or printed.

8. Compounds of the formula (III) 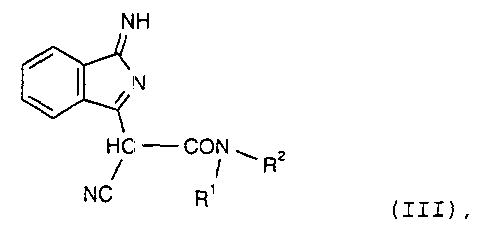 in which
R¹ represents CH₃, and
R² represents -CH₂CH₂OH or -CH₂CH₂CH₂CH₃,
or
R¹ and R², together with the N atom to which they are bonded, represent a radical.

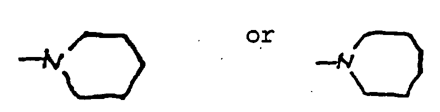

## Revendications

1. Composés de formule (I) 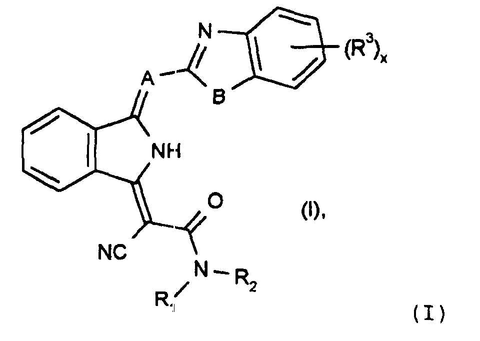 dans laquelle
A désigne N ou un radical cyanométhylène,
B désigne S, O ou NH,
R¹ désigne un radical aliphatique saturé ou insaturé ayant de 1 à 12 atomes de carbone, en particulier de 1 à 10 atomes de carbone, qui est éventuellement interrompu par un ou plusieurs atomes d'oxygène et/ou éventuellement substitué par un ou plusieurs substituants identiques ou différents parmi le groupe constitué d'un alcoxy, d'un acyloxy, d'un halogène, de CN, d'un aryle, en particulier d'un phényle,
R² représente un hydrogène ou un aryle, ou est tel que défini pour R¹, R¹ et R² étant identiques ou différents
ou
R¹ et R² forment, conjointement avec l'atome d'azote, auquel ils sont liés, un noyau hétérocyclique,
R³ représente un halogène, en particulier Cl, F et Br, un alkyle en C₁-C₄, un radical oxy aliphatique saturé ou insaturé ayant de 1 à 4 atomes de carbone, en particulier un alcoxy en C₁-C₄, qui est éventuellement substitué par un alcoxy en C₁-C₄, ou représente CN ou NO₂, et
x représente un nombre de 0 à 4.

2. Composés selon la revendication 1, qui correspondent à la formule (I) ou à ses formes tautomères
dans laquelle
x désigne 0 ou 1.

3. Composés selon la revendication 1, qui correspondent à la formule (II) ou à ses formes tautomères 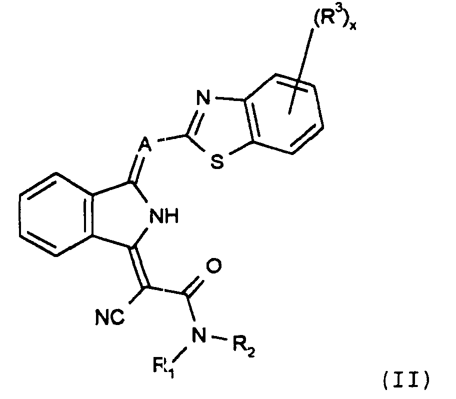 dans laquelle A, R1-R3 et x ont la signification indiquée à la revendication 1.

4. Composés selon la revendication 1, qui correspondent à la formule (I) ou à ses formes tautomères, dans laquelle R³ désigne un chlore, un méthyle, un méthoxy ou un éthoxy.

5. Procédé de préparation de composés selon la revendication 1, **caractérisés en ce que** l'on procède à la condensation d'un composé de formule (III) 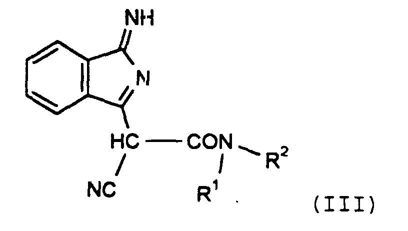 avec un composé de formule (IV) 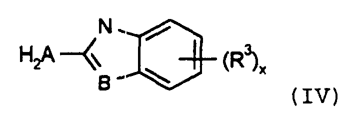 ou une amyno-isoindolénine de formule (V) 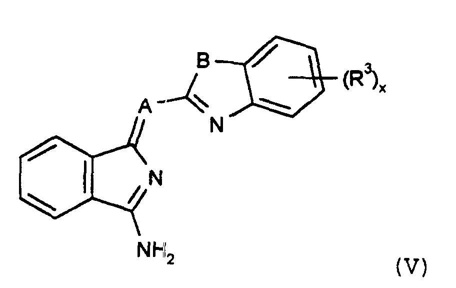 avec un cyanoacétamide de formule (VI) 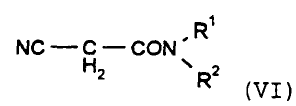 pour donner lieu à des composés de formule (I) 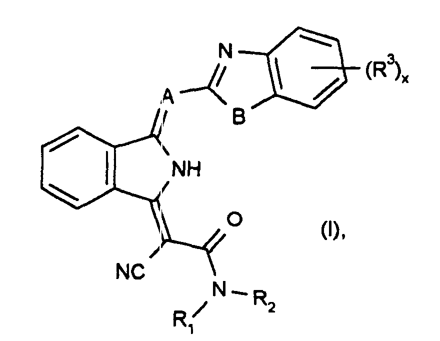 dans laquelle
A désigne N ou un radical cyanométhylène,
B désigne S, O ou NH,
R¹ désigne un radical aliphatique saturé ou insaturé ayant de 1 à 12 atomes de carbone, en particulier de 1 à 10 atomes de carbone, qui est éventuellement interrompu par un ou plusieurs atomes d'oxygène et/ou éventuellement substitué par un ou plusieurs substituants identiques ou différents parmi le groupe constitué d'un alcoxy, d'un acyloxy, d'un halogène, de CN, d'un aryle, en particulier d'un phényle,
R² représente un hydrogène ou un aryle, ou est tel que défini pour R¹, R¹ et R² étant identiques ou différents
ou
R¹ et R² forment, conjointement avec l'atome d'azote, auquel ils sont liés, un noyau hétérocyclique,
R³ représente un halogène, en particulier Cl, F et Br, un alkyle en C₁-C₄, un radical oxy aliphatique saturé ou insaturé ayant de 1 à 4 atomes de carbone, en particulier un alcoxy en C₁-C₄, qui est éventuellement substitué par un alcoxy en C₁-C₄, ou représente CN ou NO₂, et
x représente un nombre de 0 à 4.

6. Utilisation de composés selon la revendication 1, pour la teinture ou l'impression de substances à poids moléculaire élevé, entièrement ou semi-synthétiques.

7. Utilisation selon la revendication 5, **caractérisée en ce que** l'on teint ou imprime des tissus d'ameublement pour automobiles.

8. Composés de formule (III) 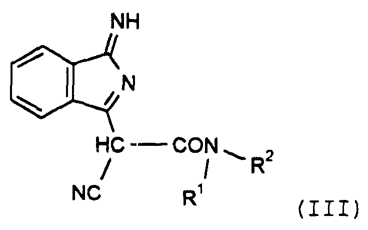 dans laquelle
R¹ désigne CH₃, et
R² désigne -CH₂CH₂OH ou -CH₂CH₂CH₂cH₃,
ou
R¹ et R² désignent, conjointement avec l'atome d'azote auquel ils sont liés, un radical
